# EUROPEAN PATENT APPLICATION

(11) **EP 4 703 479 A1**
(43) Date of publication of application: **04.03.2026**
(21) Application number: 24196438.6
(22) Date of filing: 26.08.2024
(51) Int. Cl.: C12Q 1/6804, C12Q 1/6823

(54) **A MARKER AND A METHOD FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

In a first aspect a marker (100, 200) for analysing a biological sample is provided. The marker (100, 200) comprises an affinity reagent (104) comprising an affinity reagent oligonucleotide (108) with a barcode sequence (110). The marker (100, 200) further comprises a label (112) comprising a label oligonucleotide (114) with a complementary barcode sequence (116) at least partially complementary to the barcode sequence (110) of the affinity reagent oligonucleotide (108). The marker (100, 200) further comprises at least one blocking oligonucleotide (102, 202) with a blocking sequence (118, 204) that is only partially complementary to the barcode sequence (110) of the affinity reagent oligonucleotide (108) or to the complementary barcode sequence (116) of the label oligonucleotide (114).

## Description

### Technical field

The invention relates to a marker and corresponding label, affinity reagent and kit. In a further aspect, a method for analysing a biological sample is provided.

### Background

Background fluorescence is a significant disadvantage in fluorescence imaging because it can obscure the signal from the target fluorescent molecules, reducing the contrast and clarity of generated images. The occurrence of background fluorescence in fluorescence imaging is due to several factors, including autofluorescence from biological samples and the nonspecific binding of fluorescent markers. Nonspecific binding occurs when fluorescent markers attach to unintended sites within the sample, generating additional fluorescence signals that overlap with those from targets. This overlap diminishes the signal-to-noise ratio, making it challenging to accurately detect and quantify the targets. As a result, the unwanted fluorescence can lead to inaccurate measurements, misinterpretation of data, and difficulty in distinguishing between the target and the background, thereby compromising the overall quality and reliability of the imaging results. Similar problems exist for markers which are not based on fluorescence detection, e.g. markers which are read out by sequencing as well as markers that are labelled with metal tag, a radioactive label, an enzyme tag (e.g. horseradish peroxidase), a Raman label, or gold particle for example.

Such nonspecific interactions can be influenced by factors like the concentration and chemical properties of the markers, as well as the composition and preparation of the sample or the markers. The combination of these sources of background fluorescence makes it a persistent challenge to obtain high-quality, high-contrast fluorescence images. This is particularly true for applications where a larger number of fluorescent markers is used, such as high plex applications.

### Summary

It is an object to provide a marker that enables reducing background fluorescence when analysing biological samples, in particular with a large number of target analytes.

The aforementioned object is achieved by the subject-matter of the independent claims. Advantageous embodiments are defined in the dependent claims and the following description.

In a first aspect a marker for analysing a biological sample is provided. The marker comprises an affinity reagent comprising an affinity reagent oligonucleotide with a barcode sequence. The marker further comprises a label comprising a label oligonucleotide with a complementary barcode sequence at least partially complementary to the barcode sequence of the affinity reagent oligonucleotide. The marker further comprises at least one blocking oligonucleotide with a blocking sequence that is only partially complementary to the barcode sequence of the affinity reagent oligonucleotide or to the complementary barcode sequence of the label oligonucleotide.

The only at least partially complementary blocking sequence of the at least one blocking oligonucleotide enables blocking of either the barcode sequence or the complementary barcode sequence. For example, when the components of the marker, such as the affinity reagent with the affinity reagent oligonucleotide and the label with the label oligonucleotide, are introduced into a biological sample for its analysis, the components may be introduced separately in time. In particular, the affinity reagent with the affinity reagent oligonucleotide may initially be introduced into the biological sample in order to bind the affinity reagent to a particular target analyte of the biological sample. Subsequently, the label with the label oligonucleotide may be introduced. In this case, the blocking oligonucleotide enables preventing that the affinity reagent oligonucleotide and/or the label oligonucleotide inadvertently and non-specifically hybridises to a random nucleic acid of the biological sample. A random nucleic acid may be an endogenous nucleic acid molecule or sequence that may have sequence with inadvertent similarity or complementarity to the label oligonucleotide.

Likewise, the proposed marker enables reduced binding of the label oligonucleotide to off-target affinity reagent oligonucleotides, i.e. affinity reagent oligonucleotides that are assigned to affinity reagents that are not assigned to said label oligonucleotide. Such unintended interactions that may occur between the label oligonucleotide and endogenous nucleic acids and "off-target" affinity reagent oligonucleotides may also be referred to as cross-hybridization.

In particular, the proposed marker enables a reduction in undesired unspecific binding and/or in a reduced background fluorescence. Thus, in a particular embodiment, the marker may comprise two blocking oligonucleotides, a first blocking oligonucleotide with a first blocking sequence being partially complementary to the barcode sequence of the affinity reagent oligonucleotide and a second blocking oligonucleotide with a second blocking sequence being partially complementary to the complementary barcode sequence of the label oligonucleotide.

Simultaneously, the complementarity between the barcode sequence and the complementary barcode sequence enables highly specific and stable binding between the barcode sequence and the complementary barcode sequence and therefore between the affinity reagent and the label. This avoids incorrect association of affinity reagents with markers. This is particularly relevant when a large number of different markers are used comprising affinity reagents that are specific to different target analytes, in order to identify a large number of target analytes.

The affinity reagent is preferably configured to specifically bind to a target analyte of the biological sample, for example a target protein or a target nucleic acid. For example, the affinity reagent may be an antibody, an antibody fragment, an aminoacid- or nucleic-acid-based aptamer, or a linear single stranded nucleic acid. Further preferably, the affinity reagent oligonucleotide is covalently attached to the affinity reagent.

The label comprises at least one labelling moiety, which may be configured to be optically detectable, in particular. The labelling moiety may comprise a fluorophore such as fluorescent proteins, organic or inorganic fluorescent molecules, or fluorescent nanoparticles. This renders the label detectable by an optical microscope, such as a fluorescence microscope, in particular a confocal scanning microscope, for example.

For example, the label oligonucleotide may function as a backbone for the label, which comprises the complementary barcode sequence, in particular towards a 3' or 5' end. The labelling moiety may preferably be (covalently) attached to the label oligonucleotide.

Preferably, at least two of the blocking sequences, the barcode sequence, and the complementary barcode sequence, comprise the same number of nucleotides. Thus, they are preferably of the same length.

In particular, the only partially complementary blocking sequence of the blocking oligonucleotide results in a reduced melting temperature of that blocking sequence to the respective barcode sequence of the affinity reagent oligonucleotide or complementary barcode sequence of the label oligonucleotide compared to the melting temperature of the barcode sequence to the complementary barcode sequence. This is especially the case, if the respective sequences are all the same length. The blocking sequence may only be partially complementary to the respective barcode sequence or complementary barcode sequence due to mismatched between the sequences, for example.

Thus, the hybridisation between the barcode and complementary barcode sequences is preferred compared to the hybridisation between the blocking oligonucleotide, in particular the blocking sequence, and the respective barcode or complementary barcode sequence.

For example, the number of mismatches between the blocking sequence and the respective barcode sequence or complementary barcode sequence may be in a range of 1 to 10 mismatched nucleotides for every 20nt of blocking sequence. The mismatched nucleotides are preferably uniformly distributed over the length of the blocking sequence.

Preferably, the at least one blocking oligonucleotide is separate from the affinity reagent oligonucleotide and the label oligonucleotide. In this case, the blocking oligonucleotide is not covalently linked to the affinity reagent oligonucleotide or the label oligonucleotide. The blocking oligonucleotide only binds to the respective affinity reagent oligonucleotide or label oligonucleotide by hybridisation. This enables an easy generation and assembly of the marker.

Preferably, the at least one blocking oligonucleotide is part of the affinity reagent oligonucleotide or the label oligonucleotide. Thus, the blocking oligonucleotide, and the affinity reagent oligonucleotide or the label oligonucleotide may be one continuous nucleotide sequence or nucleic acid molecule. This enables easily bringing the blocking oligonucleotide, in particular the blocking sequence, in proximity to the affinity reagent oligonucleotide or the label oligonucleotide and enabling the regular and proper hybridisation or blocking of the respective affinity reagent oligonucleotide or label oligonucleotide by the blocking oligonucleotide despite only partially complementarity or mismatches.

Preferably, the blocking sequence of the blocking oligonucleotide is a partial reverse complement of the barcode sequence of the affinity reagent oligonucleotide or of the complementary barcode sequence of the label oligonucleotide, in particular, of the respective label oligonucleotide or affinity reagent oligonucleotide the blocking oligonucleotide is a part of. This enables forming a hairpin loop between the blocking oligonucleotide and the respective barcode sequence or complementary barcode sequence. In case the marker comprises two blocking oligonucleotides, both blocking oligonucleotides may form a hairpin with the respective barcode sequence or complementary barcode sequence. Further, the blocking sequences of the two blocking oligonucleotides may be complementary to each other. Preferably, the first and second barcode sequences may be at the same distance (in terms of number of nucleotides) from the respective barcode sequence or complementary barcode sequence. This enables efficiently binding of the two blocking sequences to each other when the two blocking oligonucleotides are connected to the affinity reagent oligonucleotide or the label oligonucleotide, respectively.

Preferably, the complementary barcode sequence of the label oligonucleotide is entirely complementary to the barcode sequence of the affinity reagent, particularly at least regarding the continuous common complementary barcode sequences. Thus, there are no mismatches between the barcode sequence and the complementary barcode sequence and preferably both barcode sequences have the same number of nucleotides. This enables specific and stable binding of the label to the marker.

Preferably, a hybridised complex or duplex of the blocking sequence with the barcode sequence of the affinity reagent oligonucleotide or with the complementary barcode sequence of the label oligonucleotide has a melting temperature that is lower than a melting temperature of a hybridised complex or duplex of the barcode sequence of the affinity reagent oligonucleotide with the complementary barcode sequence of the label oligonucleotide. This enables preferential hybridisation of the barcode sequence with the complementary barcode sequence compared to the hybridisation of the blocking sequence to the respective barcode sequence or complementary barcode sequence.

Preferably, the affinity reagent oligonucleotide comprises a plurality of barcode sequences. This enables binding of a plurality of labels to the affinity reagent. Each of the barcode sequences of the plurality of the barcode sequences may be identical to each other. Preferably, the barcode sequences of the plurality differ from each other, in particular such that each label may bind to a specific predetermined one of the plurality of barcode sequences. Each barcode sequence of the plurality may be unique, for example. In a particular embodiment, a particular barcode sequence may encode detectable properties of the labelling moiety of the label that may be attached to the particular barcode sequence. The detectable properties may include excitation and emission wavelength, and emission lifetime of a fluorophore, for example.

Preferably, two, in particular all, adjacently arranged barcode sequences of the affinity reagent oligonucleotide are separated by cleavage sites configured to be - in particular selectively - cleavable by means of a cleavage agent. This enables removing barcode sequences from the affinity reagent oligonucleotide. Together with the barcode sequences, any labels bound to the respective barcode sequences are removed. This may be particularly relevant to cyclical staining methods where (different) labels are iteratively connected to the same affinity reagent, in particular between subsequent (optical) readouts. Thus, the marker enables combining a highly specific and stable binding of labels to affinity reagents and easy removal of the label from the affinity reagent.

In particular, each cleavage site may be cleavable only by a specific cleavage agent. For example, each cleavage site may be cleavable by a different enzyme, such as a restriction enzyme, to enable subsequently and specifically cleaving particular or selectable cleavage sites. Examples of cleavage sites or agents include cleavage sites or nucleotides cleavable by UV-light, restriction sites and restriction enzymes,

Preferably, the label comprises at least one labelling moiety. This enables easy detection and identification of the marker and a respective target analyte. Preferably, the labelling moiety may be covalently attached to label oligonucleotide. Alternatively, the labelling moiety may comprise an attachment oligonucleotide that may hybridise to the label oligonucleotide.

Preferably, the labelling moiety is at least one of a fluorophore, a polyyne, and a metal ion. This enables detection and identification of the marker with various detection means. Further, this enables generating a larger number of distinguishable labels.

Preferably, the barcode sequence comprises between 8 and 60 nucleotides. Similarly, the complementary barcode sequence and the blocking sequence may comprise the same number of nucleotides than the barcode sequence.

In another aspect, a system of a plurality of markers, in particular as described above, is provided. The barcode sequence of each marker of the plurality of markers differs from the barcode sequence of any other marker. In particular, each marker of the plurality of markers has a unique barcode sequence, such that labels may be attached to the affinity reagents of the markers specifically. For example, each marker of the plurality of markers has a label with detectable properties that are distinguishable from any of the remaining labels of the plurality of markers. In this case, it is desirable to attach each distinguishable label to a predetermined specific one of the affinity reagents of the plurality of markers in order to be able to associate the detection of a particular label with a particular target analyte.

In a further aspect, a label for generating a marker, in particular as described above, is provided. the label comprises a label oligonucleotide with a complementary barcode sequence being at least partially complementary to a barcode sequence of an affinity reagent oligonucleotide of an affinity reagent. The label further comprises at least one blocking oligonucleotide with a blocking sequence that is partially complementary to the barcode sequence of the label oligonucleotide. The label is configured to be connected to an affinity reagent by hybridising the barcode sequence of the affinity reagent oligonucleotide with the complementary barcode sequence of the label oligonucleotide. The label is preferably for analysing a biological sample comprising a target analyte and the affinity reagent is configured to specifically bind to the target analyte.

In a further aspect, an affinity reagent for generating a marker, in particular as described above, is provided. the affinity reagent comprises an affinity reagent oligonucleotide with a barcode sequence being at least partially complementary to a complementary barcode sequence of a label oligonucleotide of a label. The affinity reagent further comprises at least one blocking oligonucleotide with a blocking sequence that is partially complementary to the barcode sequence of the affinity reagent oligonucleotide. The affinity reagent is configured to be connected to a label by hybridising the barcode sequence of the affinity reagent oligonucleotide with the complementary barcode sequence of the label oligonucleotide. The affinity reagent is preferably for analysing a biological sample comprising a target analyte and the affinity reagent is configured to specifically bind to the target analyte.

In a further aspect, a kit for analysing a biological sample is provided. the kit comprises either a label for generating a marker, in particular as described above, and an affinity reagent with an affinity reagent oligonucleotide with a barcode sequence. The label is configured to form the marker if the barcode sequence of the affinity reagent oligonucleotide can hybridise with the complementary barcode sequence of the label oligonucleotide. Alternatively, the kit comprises an affinity reagent for generating a marker, in particular as described above, and a label with a label oligonucleotide with a complementary barcode sequence. The affinity reagent is configured to form the marker if the complementary barcode sequence of the label oligonucleotide can hybridise with the barcode sequence of the affinity reagent oligonucleotide.

In another aspect, a method for analysing a biological sample is provided. the method comprises introducing into the biological sample: at least one marker, in particular as described above; or a system of a plurality of markers, in particular as described above; or at least one label, in particular as described above, and at least one affinity reagent with an affinity reagent oligonucleotide with a barcode sequence being at least partially complementary to the complementary barcode sequence of the label oligonucleotide of the label; or at least one affinity reagent, in particular as described above, and at least one label with a label oligonucleotide with a complementary barcode sequence being at least partially complementary to the barcode sequence of the affinity reagent oligonucleotide of the affinity reagent. The method further comprises the step of generating an initial readout of the markers in the biological sample. The readout is generally generated in order to detect the markers, in particular the labels of the markers. For example, the readout may be an optical readout, in case the labels are optically detectable and comprise fluorophores as labelling moieties, for example. The readout may be generated by means of a microscope, such as a fluorescence microscope, for example.

Preferably, during the step of introducing the at least one marker, marker parts of the at least one marker are added separately. In particular, the marker parts are introduced sequentially in time. For example, the affinity reagents may initially be added and allowed to bind to their respective target analytes. Subsequently, the respective labels may be added. The marker parts may be the affinity reagent, the label, and the blocking oligonucleotide, for example.

Preferably, after generating the initial readout, a further label is introduced into the biological sample and a further readout is generated. The further label may similarly comprise a label oligonucleotide with a complementary barcode sequence that is complementary to one of the barcode sequences of an affinity reagent of one of the markers previously introduced into the biological sample. The previous labels may cleaved off and removed from the biological sample prior to introducing the further label. A further readout may be generated after introducing the further label into the biological sample. This enables providing a cyclical staining method.

Generally, a plurality of markers may be introduced into the biological sample, the markers being specific to respective target analytes or pairs of target analytes, in order to identify a large number of (different or the same) target analytes at the same time. Preferably, a target analyte is identified and/or localised within the biological sample based on the label(s) of the marker(s), in particular the labelling moieties, associated with the target analyte in the readout. Markers may be physically constituted before introducing them into the biological sample. Alternatively, affinity reagents may be barcoded with the barcode sequences and introduced into the biological sample initially and the labels comprising complementary barcode sequences may be introduced into the biological sample in a subsequent step and may then attach to their respective affinity reagent thereby forming the respective markers within the biological sample.

The system of markers, label, affinity reagent, kit and method have the same advantages as the marker. Further, the system of markers, label, affinity reagent, kit and method may be supplemented with the features of the marker described in this document, in particular, the features of the dependent claims of the marker.

### Short Description of the Figures

Hereinafter, specific embodiments are described referring to the drawings, wherein:
- Figure 1: is a schematic view of a marker comprising a blocking oligonucleotide for analysing a biological sample,

- Figure 2: is a schematic view of the marker according to Fig. 1, and
- Figure 3: is a schematic view of a marker with two blocking oligonucleotides.

### Detailed Description

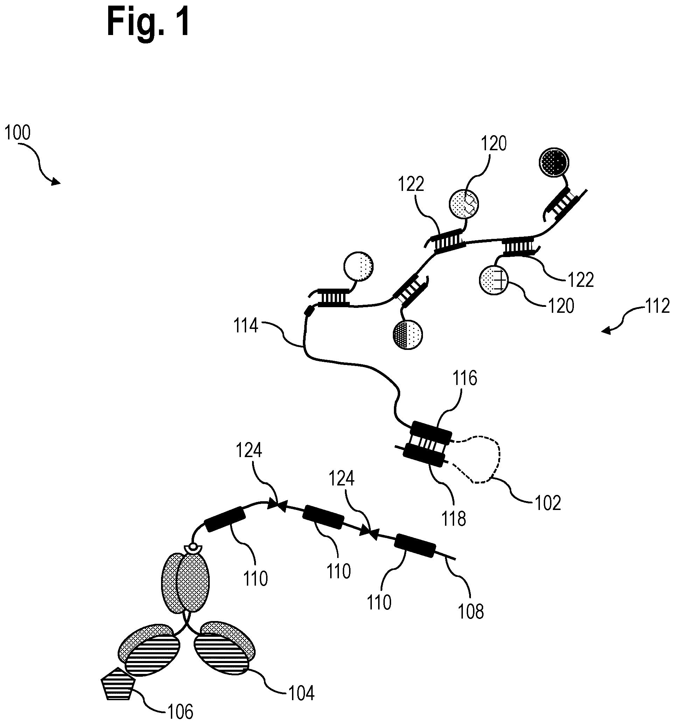

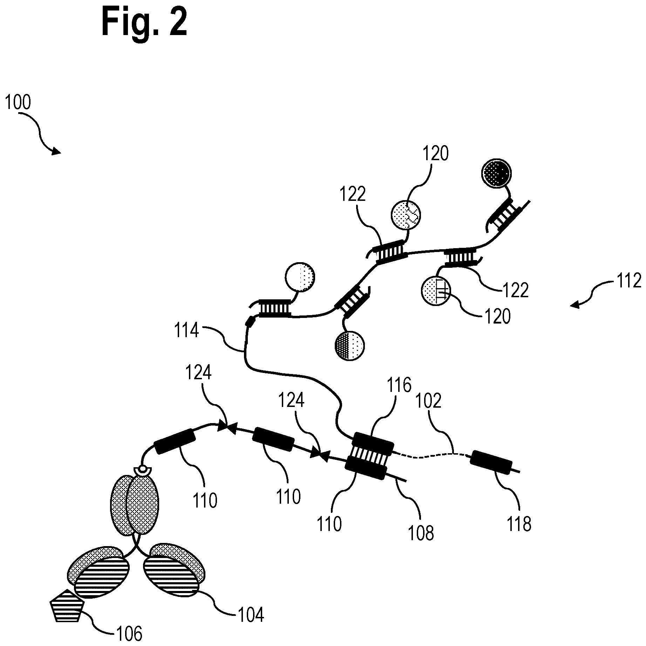

Figures 1 and 2 are schematic views of a marker 100 for analysing a biological sample (not shown). The marker 100 comprises a blocking oligonucleotide 102. The marker further comprises an affinity reagent 104 configured to specifically bind to a target analyte 106 of the biological sample. The affinity reagent 104 comprises an affinity reagent oligonucleotide 108, which is attached to the affinity reagent 104. The affinity reagent oligonucleotide 108 comprises three barcode sequences 110.

The marker 100 further comprises a label 112 that comprises the blocking oligonucleotide 102. The label further comprises a label oligonucleotide 114. The label oligonucleotide 114 comprises a complementary barcode sequence 116, which is complementary to the barcode sequence 110 of the affinity reagent oligonucleotide 108. The blocking oligonucleotide 102 and the label oligonucleotide 114 are one continuous nucleic acid molecule. The blocking oligonucleotide 102 comprises a blocking sequence 118, which is partially complementary to the complementary barcode sequence 116. Thus, the blocking sequence 118 may comprise mismatches such that it does not entirely hybridise to the complementary barcode sequence 116. Thus, the barcode sequence 110 and the complementary barcode sequence 116 preferentially hybridise to each other. Compared to that, the blocking sequence 118 and the complementary barcode sequence 116 hybridise to each other only subordinately. However, if the barcode sequence 110 is not present or in proximity to the complementary barcode sequence 116, the blocking sequence 118 may freely hybridise to the complementary barcode sequence 116.

The label 112 further comprises a plurality of labelling moieties 120. The labelling moieties 120 are (different or identical) fluorophores, for example. The labelling moieties 120 may be attached to the label oligonucleotide 114 by means of attachment oligonucleotides 122, which are complementary to respective parts of the label oligonucleotide 114.

The affinity reagent oligonucleotide 108 may comprise cleavage sites 124, which are cleavable by means of a cleavage agent (not shown). For example, the cleavage sites 124 may be restriction sites cleavable by a restriction enzyme. Preferably, each cleavage site 124 is cleavable by only one specific restriction enzyme. Alternatively, the cleavage site 124 may be a UV-sensitive nucleotide cleavable by UV-light. Cleaving one of the cleavage sites 124 enables removing the label 112 (together with the respective barcode sequence 110 of the affinity reagent oligonucleotide 108) from the affinity reagent 104.

The marker 100 comprises several barcode sequences 110. Thus, several of the labels 112 may be attached to the affinity reagent 104. Alternatively, the barcode sequences 110 may differ from each other in their specific nucleotide sequence. This enables generating labels 112 that comprise complementary barcode sequences 116 that are complementary to one of the different barcode sequences 110 and attaching these labels 112 to a specific one of the barcode sequences 110. These different labels 112 may differ from each other by the set of labelling moieties 120 that is attached to the label oligonucleotide 114. For example, each different label 112 may comprise labelling moieties 120 with different detectable properties. By attaching these labels 112 to the affinity reagent 104, cleaving the cleavage site 124, and attaching further labels 112 to another barcode sequence 110, an iterative staining method may be carried out using the marker 100.

In Figure 1, the marker 100 is shown in a state in which the blocking sequence 118 is hybridised to the complementary barcode sequence 116 and therefore, the label 112 is not (yet) bound to the affinity reagent 104. The blocking sequence 118 preferably is a reverse partial complement of the complementary barcode sequence 116. Thus, in the state according to Fig. 1, the blocking oligonucleotide 102 and the label oligonucleotide 114 may form a hairpin loop. This avoids the label 112, in particular the blocking oligonucleotide 102 or the label oligonucleotide 114, from hybridising to an off-target in the biological sample, for example. This increases the probability that the label 112 correctly binds to the affinity reagent 104, in particular its affinity reagent oligonucleotide 108, upon addition of the label 112 to the biological sample.

In Figure 2, the marker 100 is shown in a state in which the complementary barcode sequence 116 is hybridised to the barcode sequence 110 and therefore, the label 112 is bound to the affinity reagent 104. When the label 112 and the affinity reagent 104 are in proximity, the barcode sequence 110 and the complementary barcode sequence 116 preferentially hybridise to each other. Moreover, the barcode sequence 110 may displace the blocking sequence 118 from the complementary barcode sequence 116. Fig. 2 shows the marker 100 in a state, in which it may be read-out when analysing a biological sample.

In an alternative embodiment, the marker 100 may comprise the blocking oligonucleotide 102, in particular the blocking sequence 118, connected to the affinity reagent oligonucleotide 108 instead of the label oligonucleotide 114 (as shown in Figs. 1 and 2).

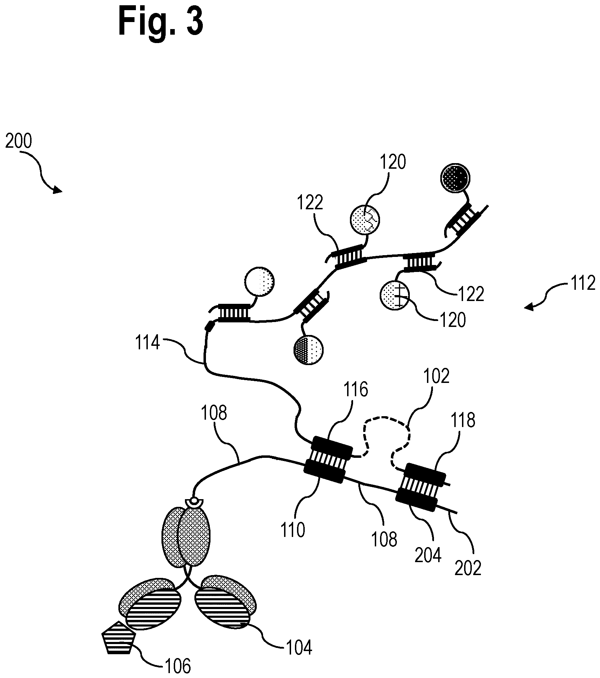

Figure 3 is a schematic view of a marker 200. Compared to the marker 100, the marker 200 comprises a second blocking oligonucleotide 202 with a second blocking sequence 204. The second blocking sequence 204 may be partially complementary to the barcode sequence 110. Preferably, the second blocking sequence 204 is entirely complementary to the blocking sequence 118.

Thus, in case of the marker 200, when the affinity reagent 104 is separate from the label 112, the second blocking sequence 204 may hybridise to the barcode sequence 110. The blocking sequence 118 hybridises to the complementary barcode sequence 116, as described for Figs. 1 and 2.

When the affinity reagent 104 and the label 112 of marker 200 are brought into proximity, for example when introducing both (sequentially) into a biological sample, the barcode sequence 110 and the complementary barcode sequence 116 preferentially hybridise to each other due to their complementarity. Similarly, the blocking sequence 118 and the second blocking sequence 204 may preferentially hybridise to each other due to their complementarity. Thus, not only do the barcode sequence 110 and the complementary barcode sequence 116 displace the respective blocking sequence 118 or second blocking sequence 204, but the blocking sequence 118 and the second blocking sequence 204 also preferentially hybridise to each other. This enables highly specific and stable binding between the label 112 and the affinity reagent 104. In particular, this avoids the label 112, in particular the blocking oligonucleotide 102 or the label oligonucleotide 114, and the affinity reagent 104, in particular the affinity reagent oligonucleotide 108, from hybridising to an off-target in the biological sample, for example. This further increases the probability that the affinity reagent 104 binds to its intended target analyte 106 in the biological sample and that the label 112 correctly binds to the affinity reagent 104, in particular its affinity reagent oligonucleotide 108, upon addition of the label 112 to the biological sample.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 200: Marker
- 102, 202: Blocking oligonucleotide
- 104: Affinity reagent
- 106: Target analyte
- 108: Affinity reagent oligonucleotide
- 110: Barcode sequence
- 112: Label
- 114: Label oligonucleotide
- 116: Complementary barcode sequence
- 118, 204: Blocking sequence
- 120: Labelling moiety
- 122: Attachment oligonucleotide
- 124: Cleavage site

## Claims

1. A marker (100, 200) for analysing a biological sample comprising:
an affinity reagent (104) comprising an affinity reagent oligonucleotide (108) with a barcode sequence (110),
a label (112) comprising a label oligonucleotide (114) with a complementary barcode sequence (116) at least partially complementary to the barcode sequence (110) of the affinity reagent oligonucleotide (108), and
at least one blocking oligonucleotide (102, 202) with a blocking sequence (118) that is partially complementary to the barcode sequence (110) of the affinity reagent oligonucleotide (108) or to the complementary barcode sequence (116) of the label oligonucleotide (114).

2. The marker according to claim 1, wherein the at least one blocking oligonucleotide (102, 202) is separate from the affinity reagent oligonucleotide (108) and the label oligonucleotide (114).

3. The marker according to claim 1, wherein the at least one blocking oligonucleotide (102, 202) is part of the affinity reagent oligonucleotide (108) or the label oligonucleotide (114).

4. The marker according to claim 3, wherein the blocking sequence (118, 204) is a partial reverse complement of the barcode sequence (110) or the complementary barcode sequence (116) of the respective oligonucleotide (108, 114) the blocking oligonucleotide (102, 202) is part of.

5. The marker according to one of the preceding claims, wherein the complementary barcode sequence (116) of the label oligonucleotide (114) is entirely complementary to the barcode sequence (110) of the affinity reagent oligonucleotide (108).

6. The marker according to one of the preceding claims, wherein a hybridised complex of the blocking sequence (118, 204) with the barcode sequence (110) of the affinity reagent oligonucleotide (108) or with the complementary barcode sequence (116) of the label oligonucleotide (114) has a melting temperature that is lower than a melting temperature of a hybridised complex of the barcode sequence (110) with the complementary barcode sequence (116).

7. The marker according to one of the preceding claims, wherein the affinity reagent oligonucleotide (108) comprises a plurality of barcode sequences (110).

8. The marker according to the preceding claim 7, wherein two adjacently arranged barcode sequences (110) of the affinity reagent oligonucleotide (108) are separated by a cleavage site (124) configured to be selectively cleavable by means of a cleavage agent.

9. The marker according to one of the preceding claims, wherein the label (112) comprises at least one labelling moiety (120).

10. The marker according to claim 9, wherein the labelling moiety (120) is at least one of a fluorophore, a polyyne, and a metal ion.

11. The marker according to one of the preceding claims, wherein the barcode sequence (110) comprises between 10 and 30 nucleotides.

12. A system of a plurality of markers according to one of the preceding claims, wherein the barcode sequence (110) of each marker (100, 200) of the plurality of markers differs from the barcode sequence (110) of any other marker (100, 200).

13. A label (112) for generating a marker (100, 200) comprising:
a label oligonucleotide (114) with a complementary barcode sequence (116) being at least partially complementary to a barcode sequence (110) of an affinity reagent oligonucleotide (108) of an affinity reagent (104), and
at least one blocking oligonucleotide (102) with a blocking sequence (118) that is partially complementary to the barcode sequence (116) of the label oligonucleotide (114),
wherein the label (112) is configured to be connected to an affinity reagent (104) by hybridising the barcode sequence (110) of the affinity reagent oligonucleotide (108) with the complementary barcode sequence (116) of the label oligonucleotide (114).

14. An affinity reagent (104) for generating a marker (100, 200) comprising:
an affinity reagent oligonucleotide (108) with a barcode sequence (110) being at least partially complementary to a complementary barcode sequence (116) of a label oligonucleotide (114) of a label (112),
at least one blocking oligonucleotide (202) with a blocking sequence (204) that is partially complementary to the barcode sequence (116) of the affinity reagent oligonucleotide (114),
wherein the affinity reagent (104) is configured to be connected to a label (114) by hybridising the barcode sequence (110) of the affinity reagent oligonucleotide (108) with the complementary barcode sequence (116) of the label oligonucleotide (114).

15. A kit for analysing a biological sample comprising:
a label (112) for generating a marker (100, 200) according to claim 13 and an affinity reagent (104) with an affinity reagent oligonucleotide (108) with a barcode sequence (110), wherein the label (112) is configured to form the marker (100, 200) if the barcode sequence (110) of the affinity reagent oligonucleotide (108) can hybridise with the complementary barcode sequence (116) of the label oligonucleotide (114), or
an affinity reagent (104) for generating a marker (100, 200) according to claim 14 and a label (112) with a label oligonucleotide (114) with a complementary barcode sequence (116), wherein the affinity reagent (104) is configured to form the marker (100, 200) if the complementary barcode sequence (116) of the label oligonucleotide (114) can hybridise with the barcode sequence (110) of the affinity reagent oligonucleotide (108).

16. A method for analysing a biological sample comprising the following steps:
introducing into the biological sample:
at least one marker (100, 200) according to one of the preceding claims 1 to 11, or
a system of a plurality of markers according to claim 12, or
at least one label (112) according to claim 13 and at least one affinity reagent (104) with an affinity reagent oligonucleotide (108) with a barcode sequence (110) being at least partially complementary to the complementary barcode sequence (116) of the label oligonucleotide (114) of the label (112), or
at least one affinity reagent (104) according to claim 14 and at least one label (112) with a label oligonucleotide (114) with a complementary barcode sequence (116) being at least partially complementary to the barcode sequence (110) of the affinity reagent oligonucleotide (108) of the affinity reagent (104); and
generating an initial readout of the markers (100, 200) in the biological sample.

17. The method according to claim 16, wherein during the step of introducing the at least one marker (100, 200), marker parts (104, 112) of the at least one marker (100, 200) are added separately.

18. The method according to one of the preceding claims 16 and 17, wherein after generating the initial readout, a further label (112) is introduced into the biological sample and a further readout is generated.
